# EUROPEAN PATENT APPLICATION

(11) **EP 4 755 879 A1**
(43) Date of publication of application: **10.06.2026**
(21) Application number: 24848216.8
(22) Date of filing: 29.07.2024
(51) Int. Cl.: C07D 401/04, C07D 211/00

(54) **METHOD FOR PREPARING ORELABRUTINIB, AND INTERMEDIATE COMPOUND THEREOF**

(30) Priority: 31.07.2023 CN 202310952107
(71) Applicant: Beijing InnoCare Pharma Tech Co., Ltd., Beijing 102206 (CN)
(72) Inventor: CHEN, Xiangyang, Beijing 102206 (CN); JIANG, Zhengguo, Beijing 102206 (CN); JIAN, Weilin, Beijing 102206 (CN)
(74) Representative: Schüssler, Andrea
(86) International application number: PCT/CN2024/108085
(87) International publication number: WO 2025/026261

(57) **Abstract**

A method for preparing orelabrutinib and an intermediate compound thereof. Specifically, the present disclosure relates to a method for preparing 6-(1-acryloylpiperidin-4-yl)-2-(phenoxyphenyl)pyridine-3-carboxamide and an intermediate compound thereof. The preparation method features simple route, inexpensive and easily available raw materials, mild conditions, and a high product yield. It can greatly reduce the production cost, is environmentally friendly, and is easily adaptable to industrial-scale production.

## Description

### Technical Field

The present disclosure relates to the field of organic compound synthesis, specifically to a method for preparing orelabrutinib, i.e., 6-(1-acryloylpiperidin-4-yl)-2-(phenoxyphenyl)pyridine-3-carboxamide, and an intermediate compound thereof.

### Background Art

Orelabrutinib, i.e., 6-(1-acryloylpiperidin-4-yl)-2-(phenoxyphenyl)pyridine-3-carboxamide of formula (V), is a Bruton's tyrosine kinase (BTK) inhibitor developed by InnoCare Pharma. It can be used for the treatment of cancer, inflammatory diseases, and autoimmune disorders (see WO2015/048662).

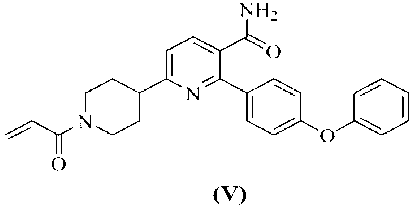

To date, there have been some reports on the methods for preparing orelabrutinib. WO2015/048662 discloses a small-scale preparation of compound V, which requires multiple palladium-catalyzed Suzuki coupling reactions to attach the piperidinyl group and the phenoxyphenyl group to the pyridine core, thereby constructing the compound of formula (V).

WO2020/173407 also discloses a method for large-scale preparation of compound V via palladium-catalyzed coupling reactions. The synthetic route is as follows:

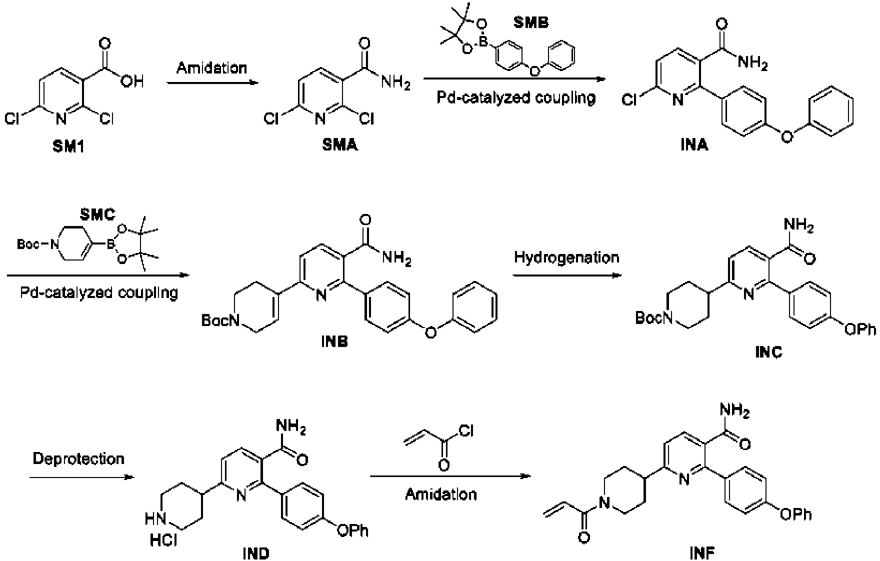

However, the above synthetic routes not only require multiple uses of expensive and environmentally unfriendly Pd catalysts, but also involve Pd-catalyzed hydrogenation under pressure, which is a hazardous process with high equipment requirements. In addition, the routes are long and the reaction steps are complex, resulting in high production costs and the generation of significant amounts of the three types of industrial waste. Therefore, further improvements are needed.

Chinese patent application CN113563305 also discloses a synthetic method, which not only requires two condensation reactions, but also involves Pd-catalyzed hydrogenation to convert a nitro group within the molecule to an amino group, followed by intramolecular cyclization and isomerization to obtain intermediate I of orelabrutinib. This method still requires the use of expensive and environmentally unfriendly Pd catalysts, and the reaction steps are complex. At the same time, the intramolecular cyclization reaction may generate different byproducts at varying temperatures, which can affect the product's purity.

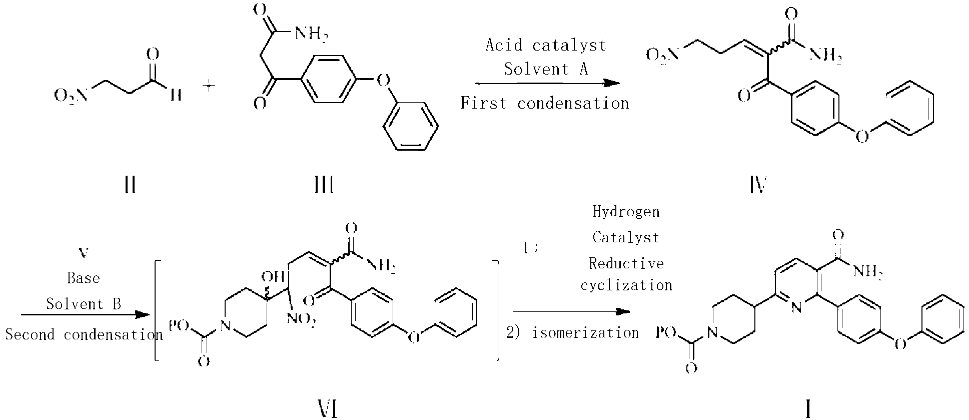

Therefore, there remains a need in the field for a synthetic method featuring ease of operation, inexpensive raw materials and suitability for the large-scale industrial production of orelabrutinib.

To overcome the drawbacks and deficiencies of the prior art, the present disclosure provides a new synthetic method for orelabrutinib. The method features ease of operation, inexpensive and readily available raw materials, high product yield, and high intermediate purity. It can reduce overall production costs to approximately 10% of those of existing methods, avoids the use of heavy metals such as Pd, is environmentally friendly, and is suitable for industrial-scale production.

### Summary of the Invention

### Definitions

In the present disclosure, the following terms have the meanings described below:
The term "alkyl", whether used alone or in combination with other groups, refers to a monovalent saturated hydrocarbon group composed of carbon and hydrogen atoms, which may be straight-chain or branched. "C₁-C₆ alkyl" refers to a straight-chain or branched alkyl group containing 1 to 6 carbon atoms, for example, methyl, ethyl, n-propyl, isopropyl, n-butyl, sec-butyl, tert-butyl, n-pentyl, isopentyl, or n-hexyl.

The term "alkenyl", whether used alone or in combination with other groups, refers to an unsaturated hydrocarbon group composed of carbon and hydrogen atoms that contains at least one double bond and may be straight-chain or branched. An alkenyl can contain 2-8 carbon atoms, for example, 2-6, 2-5, 2-4, or 2-3 carbon atoms. For instance, the term "C₂-C₆ alkenyl" refers to a straight-chain or branched alkenyl group containing 2 to 6 carbon atoms, such as ethenyl, propenyl, allyl, butenyl, or pentenyl.

The terms "alkoxy" or "alkenyloxy", whether used alone or in combination with other groups, refer to a group of the formula R^{X}-O-, where R^{X} is an alkyl or alkenyl as defined above. Representative examples of alkoxy groups include, but are not limited to, methoxy, ethoxy, propoxy (including n-propoxy and isopropoxy), butoxy (including n-butoxy, isobutoxy, and tert-butoxy), pentoxy (including n-pentoxy, isopentoxy, and neopentoxy), and hexyloxy (including n-hexyloxy and isohexyloxy). Representative examples of alkenyloxy groups include, but are not limited to, allyloxy.

The term "carbonyl", whether used alone or in combination with other groups, refers to the group -C(=O)-.

The term "halogen" refers to fluorine, chlorine, bromine, or iodine.

The term "haloalkyl" refers to an alkyl group, as described above, substituted with one or more halogen atoms, preferably a C₁-C₆ haloalkyl, such as trifluoromethyl.

The term "cycloalkyl" refers to a non-aromatic, saturated, monovalent hydrocarbon ring structure that can be a monocyclic ring, fused polycyclic ring, bridged polycyclic ring, or spirocyclic ring, having the specified number of ring atoms. A cycloalkyl group can contain 3-12 carbon atoms (i.e., C₃-C₁₂ cycloalkyl), for example 3-10, 3-8, 3-7, 3-6, or 5-6 carbon atoms. Examples of cycloalkyl groups include, but are not limited to, cyclopropyl, cyclobutyl, cyclopentyl, cyclohexyl, cycloheptyl, or cyclooctyl.

The term "aryl group" refers to a monovalent aromatic hydrocarbon group derived by removing a hydrogen atom from a single carbon atom of an aromatic ring system. Specifically, an aryl group may be a monocyclic or fused polycyclic aromatic ring structure having a specified number of ring atoms. For example, an aryl group can contain 6-14 carbon atoms, e.g., 6-10 carbon atoms, preferably 6 carbon atoms. Examples of aryl groups include C₆₋₁₄ aryl or C₆₋₁₀ aryl, such as phenyl, naphthyl, or fluorenyl

The term "aryalkyl" refers to an alkyl group, as described above, substituted with an aryl group, as described above. For example, a C₆-C₁₀ aryl-C₁₋₆ alkyl group, such as a benzyl group.

The term "heteroaryl" refers to an aromatic ring structure, either monocyclic or fused polycyclic, containing one or more (for example, 1, 2, 3, or 4) heteroatoms independently selected from O, N, and S, and having a specified number of ring atoms. The heteroaryl group may also include its N-oxide, S-oxide, or S-dioxide forms. Specifically, the aromatic ring structure can have 5-10 ring members. Examples of heteroaryl groups include 5- or 6-membered monocyclic rings, or fused bicyclic structures such as two fused 6-membered rings, two fused 5-membered rings, a fused 6-membered and 5-membered ring, or a fused 5-membered and 4-membered ring. Heteroaryl rings typically contain up to 4 heteroatoms, more commonly up to 3 heteroatoms, and more typically up to 2 heteroatoms, for example, a single heteroatom independently selected from O, N, and S, where N and S may be in oxidized forms such as N-oxide, S=O, or S(O)₂. In one embodiment, the heteroaryl ring contains at least one ring nitrogen atom, at least one ring sulfur atom, or at least one ring oxygen atom. For example, the heteroaryl group can be a fused ring system containing 1, 2, 3, or 4 heteroatoms independently selected from N, O, or S, such as benzofuran, benzothiophene, indole, benzimidazole, indazole, benzotriazole, pyrrolo[2,3-b]pyridine, pyrrolo[2,3-c]pyridine, pyrrolo[3,2-c]pyridine, pyrrolo[3,2-b]pyridine, imidazo[4,5-b]pyridine, imidazo[4,5-c]pyridine, pyrazolo[4,3-d]pyridine, pyrazolo[4,3-c]pyridine, pyrazolo[3,4-c]pyridine, pyrazolo[3,4-b]pyridine, isoindole, purine, midazolinoindole, imidazo[1,2-a]pyridine, imidazo[1,5-a]pyridine, pyrazolo[1,5-a]diazine, pyrrolo[1,2-b]pyrimidine, imidazo[1,2-c]pyrimidine, 5H-pyrrolo[3,2-b]pyrazine, 1H-pyrazolo[4,3-b]pyrazine, 1H-pyrazolo[3,4-d]pyrimidine, 7H-pyrrolo[2,3-d]pyrimidine, quinoline, isoquinoline, cinnoline, quinazoline, quinoxaline, phthalazine, 1,6-naphthyridine, 1,7-naphthyridine, 1,8-naphthyridine, 1,5-naphthyridine, 2,6-naphthyridine, 2,7-naphthyridine, pyrido[3,2-d]pyrimidine, pyrido[4,3-d]pyrimidine, pyrido[3,4-d]pyrimidine, pyrido[2,3-d]pyrimidine, pyrido[2,3-b]pyrazine, pyrido[3,4-b]pyrazine, pyrimido[5,4-d]pyrimidine, pyrazino[2,3-b]pyrazine, and pyrimido[4,5-d]pyrimidine. For example, the heteroaryl group can be a 5- or 6-membered heteroaryl containing 1 or 2 heteroatoms independently selected from N, O, or S. Suitable examples of 5-membered monocyclic heteroaryl groups include, but are not limited to, pyrrolyl, furyl, thienyl, imidazolyl, furazanyl, oxazolyl, oxadiazolyl, triazolyl, isoxazolyl, thiazolyl, isothiazolyl, pyrazolyl, triazolyl, and tetrazolyl groups. Suitable examples of 6-membered monocyclic heteroaryl groups include, but are not limited to, pyridyl, pyrazinyl, diazinyl, pyrimidinyl, and triazinyl groups. The atom of the heteroaryl group that is connected to the rest of the compound can be either a carbon atom or a heteroatom, as long as it is chemically feasible.

The term "heterocyclic group" refers to a non-aromatic saturated ring structure, which may be monocyclic, fused polycyclic, spirocyclic, or bridged polycyclic, containing one or more (for example, 1, 2, 3, or 4) heteroatoms independently selected from O, N, and S, and having a specified number of ring atoms. The heterocyclic group may also include its N-oxide, S-oxide, or S-dioxide forms. A heterocyclic group can have 3-12 ring members (referred to as 3-12-membered heterocycloalkyl), for example, 3-10, 3-8, 3-7, 4-8, 4-7, 4-6, or 5-6 ring members. Heterocyclic groups typically contain no more than 4 heteroatoms (for example, 1, 2, 3, or 4 heteroatoms). Suitable examples of heterocyclic groups include, but are not limited to, azetidinyl, oxetanyl, thietanyl, pyrrolidinyl (e.g., 1-pyrrolidinyl, 2-pyrrolidinyl, and 3-pyrrolidinyl), tetrahydrofuranyl (e.g., 1-tetrahydrofuranyl, 2-tetrahydrofuranyl, and 3-tetrahydrofuranyl), tetrahydrothiophenyl (e.g., 1-tetrahydrothiophenyl, 2-tetrahydrothiophenyl, and 3-tetrahydrothiophenyl), piperidinyl (e.g., 1-piperidinyl, 2-piperidinyl, 3-piperidinyl, and 4-piperidinyl), tetrahydropyranyl (e.g., 4-tetrahydropyranyl), tetrahydrothiopyranyl (e.g., 4-tetrahydrothiopyranyl), morpholinyl, thiamorpholinyl, dioxanyl, piperazinyl, or azepanyl, diazepanyl (e.g., 1,4-diazepanyl), or 3,6-diazabicyclo[3.1.1]heptyl, or 3-azabicyclo[3.2.1]octyl. The atom of the heterocyclic group that is connected to the rest of the compound can be either a carbon atom or a heteroatom, provided it is chemically feasible.

The term "heterocyclic group" also includes the above-defined heterocyclic groups containing at least one (for example, 1, 2, or 3) double bond. Examples include pyrroline groups (e.g., 1-pyrroline, 2-pyrroline, 3-pyrroline, 4-pyrroline, or 5-pyrroline), dihydrofuranyl groups (e.g., 1-dihydrofuranyl, 2-dihydrofuranyl, 3-dihydrofuranyl, 4-dihydrofuranyl, or 5-dihydrofuranyl), dihydrothiophenyl groups (e.g., 1-dihydrothiophenyl, 2-dihydrothiophenyl, 3-dihydrothiophenyl, or 4-dihydrothiophenyl), tetrahydropyridinyl groups (e.g., 1-, 2-, 3-, 4-, 5-, or 6-tetrahydropyridinyl), tetrahydropyranyl groups (e.g., 4-tetrahydropyranyl), or tetrahydrothiopyranyl groups (e.g., 4-tetrahydrothiopyranyl).

The term "acyl" refers to a group of the formula R^{y}-C(=O)-, where R^{y} is an alkyl, alkenyl, cycloalkyl, aryl, arylalkyl, heteroaryl, or heterocyclic group, as defined above.

The term "substituted or unsubstituted" means that the group may be unsubstituted or substituted with one or more (for example, 1, 2, 3, 4, 5, or more) substituents, which may be the same or different.

The terms "substituted or unsubstituted C₁₋₆ alkyl", "substituted or unsubstituted C₃-C₈ cycloalkyl", "substituted or unsubstituted C₆-C₁₀ aryl", "substituted or unsubstituted C₅-C₁₀ heteroaryl", "substituted or unsubstituted 3-12-membered heterocyclic group", "substituted or unsubstituted C₃-C₈ cycloalkyl-C₁₋₆ alkyl", "substituted or unsubstituted C₆-C₁₀ aryl-C₁₋₆ alkyl", "substituted or unsubstituted C₅-C₁₀ heteroaryl-C₁₋₆ alkyl", or "substituted or unsubstituted 3-12-membered heterocyclic-C₁₋₆ alkyl" refer, respectively, to the above-described alkyl, cycloalkyl, aryl, heteroaryl, heterocyclic, cycloalkylalkyl, arylalkyl, heteroarylalkyl, or heterocyclic-alkyl groups, which may optionally be substituted with one or more of the substituents described above, such as alkyl, alkoxy, halogen, haloalkyl, nitro, cyano, hydroxyl, and/or amino groups. Their examples include, but are not limited to, 2-hydroxyethyl, 2-aminoethyl, 3-hydroxypropyl, 4-hydroxybutyl, methylcyclohexyl, 2-methylphenyl, 4-methylphenyl, 4-propylbenzyl, 4-methylbenzyl, 3-chloropropyl, 4-chlorobenzyl, and 4-nitrobenzyl.

The term "C₁-C₄ alcohol" refers to an alcohol containing 1-4 carbon atoms, such as methanol, ethanol, n-propanol, isopropanol, n-butanol, isobutanol, or tert-butanol.

The term "about", when used in conjunction with a numerical value, refers to all values within a range that is 10% below to 10% above the specified value.

### Embodiments

The purpose of the present disclosure is to provide a new method for preparing a compound of formula (V). This method features simple route, inexpensive and easily available raw materials, mild conditions, a high product yield, and high intermediate purity. It can greatly reduce the production cost, is environmentally friendly, and is easily adaptable to industrial-scale production.

In a first aspect, the present disclosure provides a method for preparing a compound of formula (III-3), 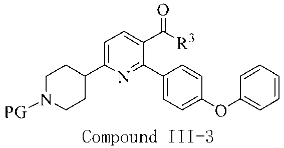 which comprises:
(a) letting a compound of formula (I) and a compound of formula (II) undergo a cyclization reaction with ammonia or an ammonium salt in the presence of an acid to obtain a compound of formula (III-3), 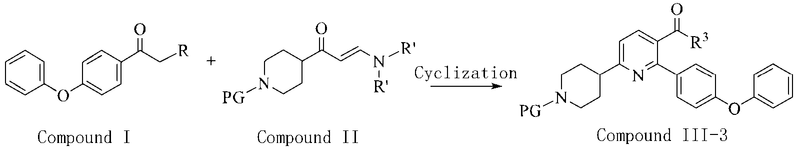 where
   R is -CN, -C(O)NH2, -C(O)N(R¹)₂, or -C(O)OR²,
   R³ is -NH₂, -N(R¹)₂, or -OR²,
   PG represents an amino protecting group, such as acyl, alkyl, arylalkyl, alkoxycarbonyl, or arylalkoxycarbonyl groups, for example, benzyl, tert-butoxycarbonyl, benzyloxycarbonyl, fluorenylmethoxycarbonyl, or allyloxycarbonyl, preferably tert-butoxycarbonyl;
   each R' is independently a substituted or unsubstituted C₁₋₆ alkyl, or R' together with the nitrogen atom to which it is attached forms a substituted or unsubstituted 3-8-membered heterocyclic group;
   each R¹ is independently H [sic: hydrogen] or an amino protecting group, such as acyl, alkyl, arylalkyl, alkoxycarbonyl, or arylalkoxycarbonyl, for example, benzyl, tert-butoxycarbonyl, benzyloxycarbonyl, fluorenylmethoxycarbonyl, or allyloxycarbonyl, preferably tert-butoxycarbonyl; with the condition that the two R¹ groups attached to the same nitrogen atom are not simultaneously hydrogen; and
   each R² is independently a substituted or unsubstituted C₁₋₆ alkyl, substituted or unsubstituted C₃-C₈ cycloalkyl, substituted or unsubstituted C₆-C₁₀ aryl, substituted or unsubstituted C₅-C₁₀ heteroaryl, substituted or unsubstituted 3-12-membered heterocyclic group, substituted or unsubstituted C₃-C₈ cycloalkyl-C₁₋₆ alkyl,
   substituted or unsubstituted C₆-C₁₀ aryl-C₁₋₆ alkyl, substituted or unsubstituted C₅-C₁₀ heteroaryl-C₁₋₆ alkyl, or substituted or unsubstituted 3-12-membered heterocyclic-C₁₋₆ alkyl group;
      or
(b) letting a compound of formula (VI) and a compound of formula (VII) undergo a cyclization reaction with ammonia or an ammonium salt in the presence of an acid to obtain a compound of formula (III-3), 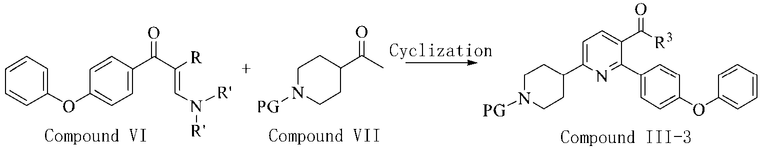 where R, R¹, R², R³, PG, and R' are as defined above.

In one embodiment, the present disclosure provides a method for preparing a compound of formula (III), 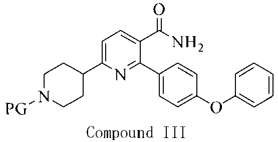 which comprises:
(A) first performing the cyclization reaction as described above in step (a), followed by:
   (A1) when R is -CN or -C(O)NH₂, the cyclization reaction directly affords a compound of formula (III), with the compound of formula (III) corresponding to a compound of formula (III-3) in which R³ is -NH₂, 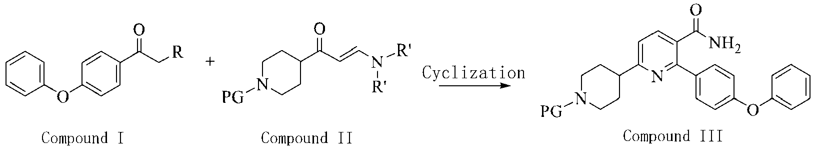
   (A2) when R is -C(O)OR², after the cyclization reaction, the resulting ester intermediate III' can be converted to a compound of formula (III) via aminolysis with an amidation reagent, or the ester intermediate III' can first be hydrolyzed in a basic solution to the corresponding acid, followed by condensation with ammonia or an ammonium salt to obtain a compound of formula (III), 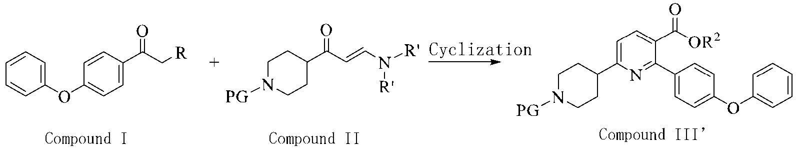 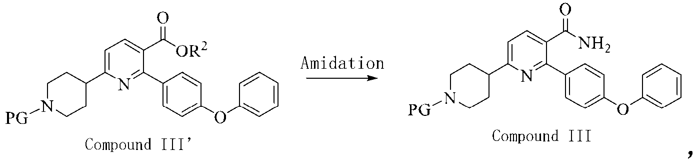 or
   (A3) when R is -C(O)N(R¹)₂, after the cyclization reaction, the compound III" is converted to a compound of formula (III) via a deprotection reaction, 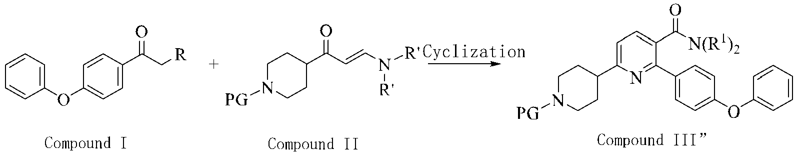 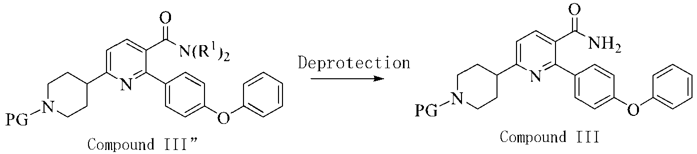 where R, R¹, R², R³, PG, and R' are as defined above; or
(B) first performing the cyclization reaction as described above in step (b), followed by:
   (B1) when R is -CN or -C(O)NH₂, the cyclization reaction directly affords a compound of formula (III), with the compound of formula (III) corresponding to a compound of formula (III-3) in which R³ is -NH₂, 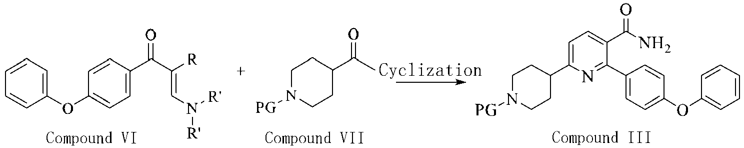
   (B2) when R is -C(O)OR², after the cyclization reaction, the resulting ester intermediate III' can be converted to a compound of formula (III) via aminolysis with an amidation reagent, or the ester intermediate III' can first be hydrolyzed in a basic solution to the corresponding acid, followed by condensation with ammonia or an ammonium salt to obtain a compound of formula (III), 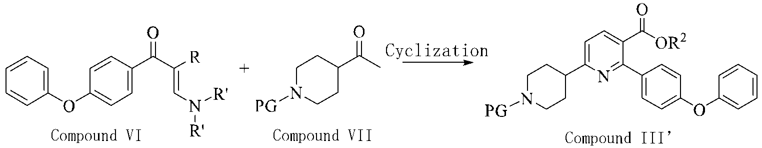 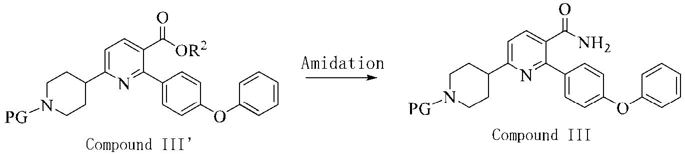
   (B3) when R is -C(O)N(R¹)₂, after the cyclization reaction, the compound III" is converted to a compound of formula (III) via a deprotection reaction, 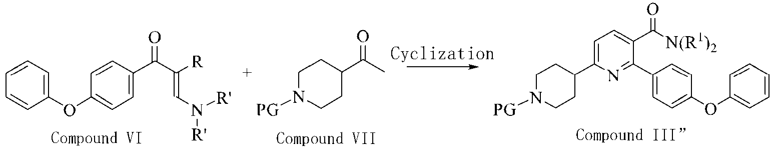 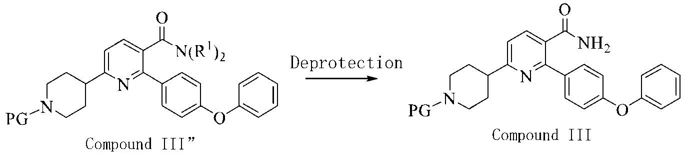 where R, R¹, R², R³, PG, and R' are as defined above.

In a preferred embodiment, the ammonium salt includes salts formed with inorganic or organic acids, such as ammonium formate, ammonium acetate, ammonium oxalate, ammonium chloride, ammonium sulfate, ammonium nitrate, ammonium methanesulfonate, and ammonium trifluoroacetate.

In a preferred embodiment, the acid includes organic and inorganic acids, such as formic acid, acetic acid, trifluoroacetic acid, hydrochloric acid, sulfuric acid, methanesulfonic acid, oxalic acid, and p-toluenesulfonic acid.

In a preferred embodiment, compound I, compound II, ammonia or an ammonium salt, and an acid react in a single organic solvent or a mixed solvent system to produce compound III.

In another preferred embodiment, compound VI, compound VII, ammonia or an ammonium salt, and an acid react in a single organic solvent or a mixed solvent system to produce compound III.

The reaction solvent is selected from commonly used organic solvents, such as methanol, ethanol, trifluoroethanol, n-propanol, isopropanol, hexafluoroisopropanol, n-butanol, acetonitrile, tetrahydrofuran, toluene, or any mixture of two or more of these solvents.

The reaction temperature ranges from 40 to 120°C, preferably from 60 to 90°C, for example, from 60 to 75°C or from 75 to 90°C, and more preferably is 75°C.

The molar ratio of ammonium salt to acid is from 0.80/1.00 to 1.20/0.80, preferably 0.95/1.00, 1.00/1.05, or 1.00/1.15, and more preferably 1.00/1.05 or 1.00/1.15. The reaction time is typically 10 to 24 hours.

In a preferred embodiment, when R is -C(O)OR², the amidation reagent includes ammonia or an ammonium salt, such as ammonium chloride, ammonium formate, ammonium acetate, ammonium oxalate, ammonium sulfate, ammonium nitrate, ammonium methanesulfonate, or ammonium trifluoroacetate.

In a preferred embodiment, when R is -C(O)OR², the base solution includes alkali or alkaline earth metal hydroxides, carbonates, bicarbonates, or alkoxides, such as the hydroxides, carbonates, or alkoxides of lithium, sodium, potassium, or calcium, for example, sodium tert-butoxide, potassium tert-butoxide, lithium hydroxide, sodium hydroxide, potassium hydroxide, sodium carbonate, or potassium carbonate.

In a preferred embodiment, when R is -C(O)N(R¹)₂, the method for deprotecting the amino protecting group is well known to those skilled in the art, for example, as described in Protective Groups in Organic Synthesis, Green and Wuts, 1999, ISBN: 9780471160199.

In a second aspect, the present disclosure provides a method for preparing a compound of formula (V), which comprises:
Step 1: performing step (A) or (B) as described above to obtain a compound of formula (III);
Step 2: deprotecting the compound of formula (III) to obtain a compound of formula (IV); 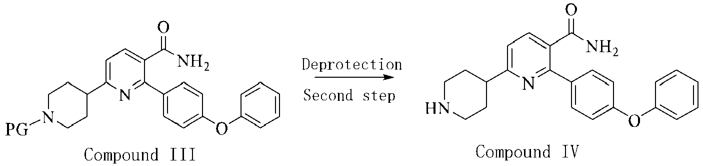 and
Step 3:letting the compound of formula (IV) undergo a reaction with acryloyl chloride, or with 3-chloropropionyl chloride followed by HCl elimination under basic conditions, thereby obtaining a compound of formula (V), 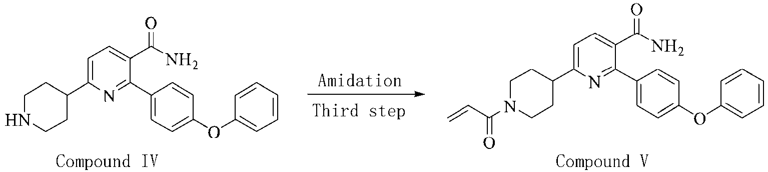 where R, R¹, R², PG, and R' are as defined above.

In another embodiment, the present disclosure provides a method for preparing a compound of formula (V), which comprises:
Step 1: performing step (A) or (B) as described above to obtain a compound of formula (III");
Step 2: removing the PG and R¹ groups from the compound of formula (III") to obtain a compound of formula (IV), 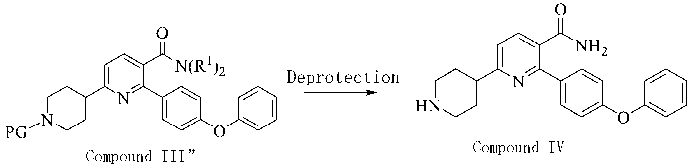 and
Step 3:letting the compound of formula (IV) undergo a reaction with acryloyl chloride, or with 3-chloropropionyl chloride followed by HCl elimination under basic conditions, thereby obtaining a compound of formula (V). 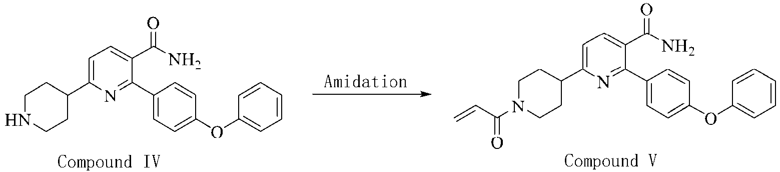
Alternatively, after step 1, perform steps 2', 3', and 4' as follows:
Step 2': deprotecting the compound of formula (III") to obtain a compound of formula (IV'); 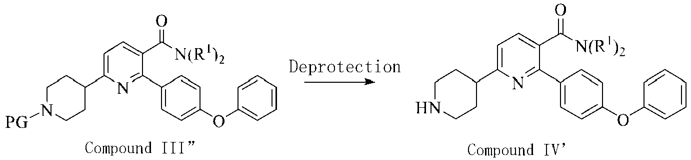
Step 3': letting the compound of formula (IV') undergo a reaction with acryloyl chloride, or with 3-chloropropionyl chloride followed by HCl elimination under basic conditions, thereby obtaining a compound of formula (V'), 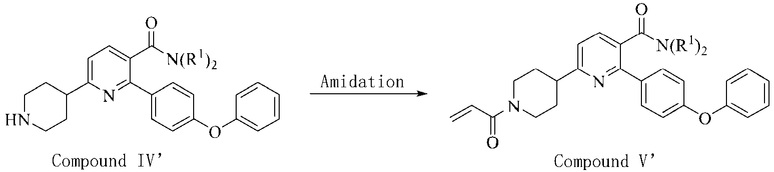
Step 4': removing the R¹ group from the compound of formula (V') to obtain a compound of formula (V), 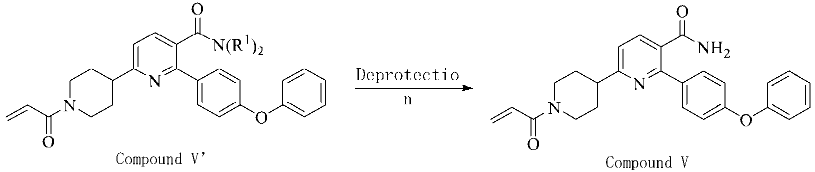 where R, R¹, R², PG, and R' are as defined above.

In another embodiment, the present disclosure provides a method for preparing a compound of formula (V), which comprises:
Step 1": performing step (A) or (B) as described above to obtain a compound of formula (III");
Step 2": removing the PG group from the compound of formula (III') to obtain a compound of formula (IV"), 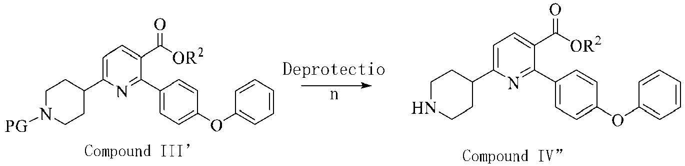
Step 3": letting the compound of formula (IV") undergo a reaction with acryloyl chloride, or with 3-chloropropionyl chloride followed by HCl elimination under basic conditions, thereby obtaining a compound of formula (V"), 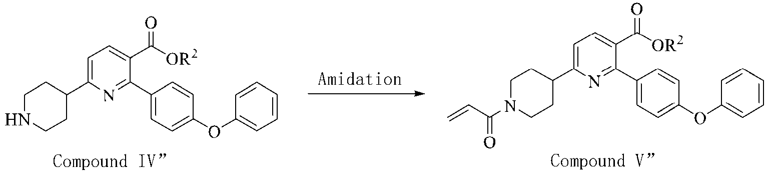
Step 4": amidation of the -OR² group of the compound of formula (V") to obtain a compound of formula (V), 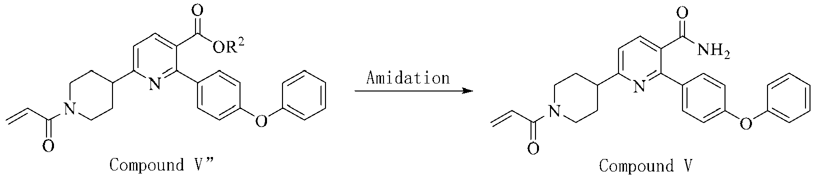 where R, R¹, R², PG, and R' are as defined above.

In a preferred embodiment, the amidation of the above -OR² group is carried out using an amidation reagent comprising ammonia or an ammonium salt, such as ammonium chloride, ammonium formate, ammonium acetate, ammonium oxalate, ammonium sulfate, ammonium nitrate, ammonium methanesulfonate, or ammonium trifluoroacetate.

In a particularly preferred embodiment, the present disclosure relates to a method for preparing a compound of formula (V) as follows: 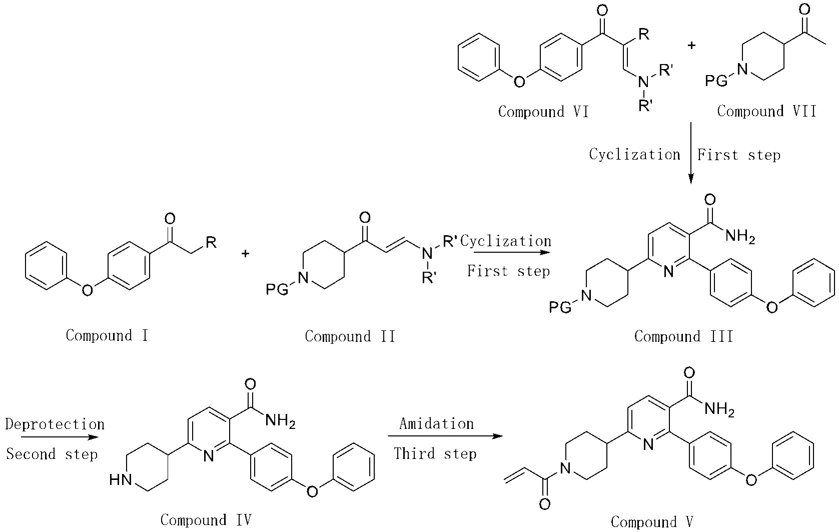
where R, R¹, R², PG, and R' are as defined above, and
wherein reaction conditions for the first, second, and third steps are as defined for the corresponding reactions described above.

In a preferred embodiment, when PG is a tert-butoxycarbonyl group, compound III is deprotected in a hydrochloric acid solution to obtain the hydrochloride salt of compound IV.

In another preferred embodiment, when PG is a benzyl or benzyloxycarbonyl protecting group, compound III is deprotected by hydrogenation using a palladium catalyst to obtain compound IV.

In another preferred embodiment, compound IV or its hydrochloride salt undergoes a reaction with acryloyl chloride or 3-chloropropionyl chloride under basic conditions to obtain 6-(1-acryloylpiperidin-4-yl)-2-(phenoxyphenyl)pyridine-3-carboxamide (compound V).

In a more preferred embodiment, in the second step, when PG is Boc, hydrochloric acid gas or an organic solution containing hydrochloric acid (for example, a solution of hydrochloric acid in ethyl acetate or a C₁-C₄ alcohol such as ethanol) is added to a solution of compound III in an organic solvent (for example, compound III in a C₁-C₄ alcohol such as ethanol or in dichloromethane), or a solution of compound III in an organic solvent is added to the hydrochloric acid solution for Boc deprotection to obtain the hydrochloride salt of compound IV.

In another more preferred embodiment, in the second step, when PG is Boc, hydrochloric acid gas, hydrochloric acid, or an organic solution containing hydrochloric acid (for example, a solution of hydrochloric acid in ethyl acetate or a C₁-C₄ alcohol such as ethanol) is added to a solution of compound III in an organic solvent (for example, compound III in a C₁-C₄ alcohol such as ethanol or acetone, or in dichloromethane), or a solution of compound III in an organic solvent is added to the hydrochloric acid solution for Boc deprotection to obtain the hydrochloride salt of compound IV.

In another more preferred embodiment, in the second step, when PG is a benzyl or benzyloxycarbonyl group, compound IV is obtained by deprotection using a palladium catalyst under hydrogen. The palladium catalyst may include palladium on carbon, palladium hydroxide on carbon, palladium chloride, triphenylphosphine palladium chloride, palladium acetate, and the like. After the formation of compound IV, a hydrochloric acid solution can be added to VI to generate the hydrochloride salt of compound IV.

In another more preferred embodiment, in the third step, compound IV or its hydrochloride salt, acryloyl chloride, undergoes a reaction with a base in a single or mixed solvent (pH 8-14) at a low temperature (0-8°C) to produce compound V. The solvent may be an organic solvent (for example, THF or dichloromethane) or a mixture of an organic solvent and water. The base may be an inorganic salt such as a bicarbonate, carbonate, phosphate, or hydroxide. The reaction time is typically 1-5 hours or 1-3 hours.

Alternatively, in another more preferred embodiment, in the third step, compound IV or its hydrochloride salt is first reacted with 3-chloropropionyl chloride in a moderately basic solution (pH 10-12, for example, a carbonate or phosphate solution) to form an intermediate, and then a strongly basic solution (for example, DBU or a hydroxide solution) is added to raise the pH to 14, thereby generating compound V. The solvent may be an organic solvent, either with or without water (for example, THF or dichloromethane).

### Specific Embodiments

### Embodiments:

### Embodiments 1-3 are shown in Scheme 1.

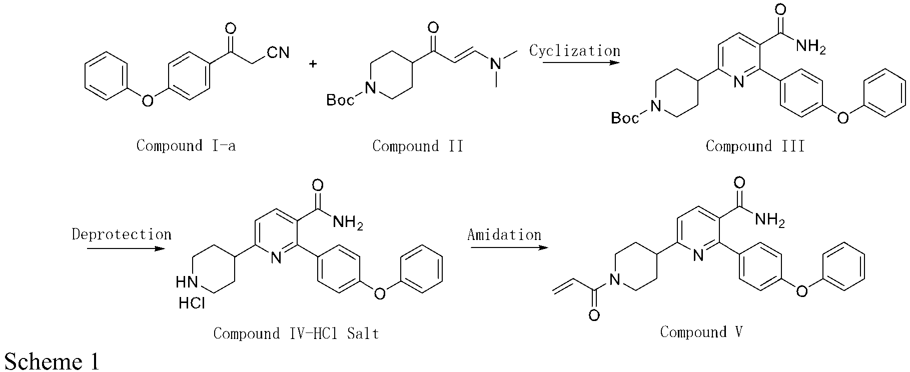

### Embodiment 1. Preparation of compound III

Compound I-a (8.00 g, 33.7 mmol), compound II (10.52 g, 37.2 mmol), ammonium acetate (10.40 g, 135 mmol), ethanol (120 mL, 15 v/v), and acetonitrile (120 mL, 15 v/v) were sequentially added to a reaction vessel. Mechanical stirring was started, and methanesulfonic acid (13.28 g, 138 mmol) was added. The mixture was heated to 80°C and maintained for approximately 24 h. The reaction mixture was cooled to 30°C and concentrated under reduced pressure until essentially no distillate was collected. Purified water (50.0 g, 6.25 w/w), toluene (100 mL, 12.5 v/v), and dichloromethane (50 mL, 12.5 v/v) were added for liquid-liquid extraction. The organic phase was washed with 5% aqueous sodium chloride solution (50.0 g, 6.25 w/w) and separated. n-Heptane (100 mL, 12.5 v/v) was added, and the mixture was heated to 70°C to concentrate under atmospheric pressure and induce crystallization. The mixture was then slowly cooled to 10°C for 4.0 h and maintained at 10°C for 2.0 h before filtration. The filter cake was dried under reduced pressure to obtain compound III (10.00 g, 62.5% yield, 98.9% purity).

¹H NMR (400 MHz, DMSO-*d*₆) δ ppm 1.41 (s, 9 H), 1.55 - 1.73 (m, 2 H), 1.86 (br d, J=10.76 Hz, 2 H), 2.74 - 3.07 (m, 3 H), 4.08 (br d, *J*=11.49 Hz, 2 H), 6.99 - 7.12 (m, 4 H), 7.14 - 7.23 (m, 1 H), 7.29 (d, J=7.82 Hz, 1 H), 7.40 - 7.46 (m, 2 H), 7.48 (s, 1 H), 7.69 - 7.76 (m, 3 H), 7.85 (s, 1 H).

The preparation of compound I-a can be found in J. Am. Chem. Soc. 2011, 133(4), 774-776. Compound I-a was synthesized in a two-step chemical reaction using 4-phenoxybenzoic acid SM1 (purchased from Bide Pharmatech Co., Ltd.) as the raw material.

The preparation of compound II follows the reference, Org. Lett. 2023, 25(25), 4688-4693. Compound II was synthesized in a one-step chemical reaction using 1-tert-butoxycarbonyl-4-acetylpiperidine (VII) (purchased from Bide Pharmatech Co., Ltd.) as the raw material.

### Embodiment 2. Preparation of the hydrochloride salt of compound IV

Compound III (8.00 g, 16.9 mmol) and dichloromethane (40 mL, 5 v/v) were added to a reaction vessel, and mechanical stirring was started to obtain a clear solution, which was transferred to a dropping funnel. Hydrochloric acid in ethanol solution (40 mL, 30 wt%, 5 v/v) and anhydrous ethanol (40 mL, 5 v/v) were added to the reaction vessel, and the mixture was cooled to 0°C. The solution in the dropping funnel was added dropwise within approximately 1 h, and the mixture was then heated to 30°C and maintained for about 12 h of reaction. The mixture in the reaction vessel was concentrated under reduced pressure until essentially no distillate was collected. Methanol (60 mL, 7.5 v/v) and ethyl acetate (60 mL, 7.5 v/v) were added sequentially, and the mixture was crystallized at 10°C. After maintaining at 10°C for 2.0 h, the mixture was filtered, and the filter cake was dried under reduced pressure to obtain the hydrochloride salt of compound IV (6.92 g, 94% yield, 99.1% purity).

¹H NMR (400 MHz, DMSO-*d*₆) δ ppm 1.98 - 2.15 (m, 4 H), 2.93 - 3.07 (m, 2 H), 3.12 (tt, *J*=10.21, 5.07 Hz, 1 H), 3.35 (br d, *J*=12.47 Hz, 2 H), 7.00 - 7.13 (m, 4 H), 7.14 - 7.26 (m, 1 H), 7.33 (d, J=8.07 Hz, 1 H), 7.38 - 7.49 (m, 2 H), 7.54 (br s, 1 H), 7.66 - 7.78 (m, 2 H), 7.84 (d, J=7.83 Hz, 1 H), 7.92 (br s, 1 H), 8.99 (br d, *J=10.03* Hz, 1 H), 9.35 (br d, J=10.51 Hz, 1 H).

### Embodiment 2'. Preparation of the hydrochloride salt of compound IV

Compound III (8.00 g, 16.9 mmol) and acetone (80 mL, 10 v/v) were added to a reaction vessel, and stirring was started. Concentrated hydrochloric acid (9.20 g, 36-38 wt%, 1.15 w/w) was then added to the reaction vessel, and the temperature in the reaction vessel was controlled at 20-30°C for about 8 h of reaction. Acetone (80 mL, 10 v/v) was added, and the mixture was stirred at 25°C for 2 h. The solid was filtered, and the filter cake was washed with acetone (32 mL, 4 v/v) and dried to obtain the hydrochloride salt of compound IV (6.92 g, 94% yield, 99.8% purity).

¹H NMR (400 MHz, DMSO-*d*₆) δ ppm 1.98 - 2.15 (m, 4 H), 2.93 - 3.07 (m, 2 H), 3.12 (tt, J=10.21, 5.07 Hz, 1 H), 3.35 (br d, *J=12.47* Hz, 2 H), 7.00 - 7.13 (m, 4 H), 7.14 - 7.26 (m, 1 H), 7.33 (d, J=8.07 Hz, 1 H), 7.38 - 7.49 (m, 2 H), 7.54 (br s, 1 H), 7.66 - 7.78 (m, 2 H), 7.84 (d, J=7.83 Hz, 1 H), 7.92 (br s, 1 H), 8.99 (br d, *J=10.03* Hz, 1 H), 9.35 (br d, J=10.51 Hz, 1 H).

### Embodiment 3. Preparation of compound V

Hydrochloride salt of compound IV (4.00 g, 9.76 mmol), tetrahydrofuran (64 mL, 16 v/v), and purified water (64 mL, 16 v/v) were added to a reaction vessel. Mechanical stirring was started, and sodium bicarbonate (4.76 g, 56.7 mmol) was added in portions. The mixture was cooled to 0°C, and a pre-prepared solution of 3-chloropropionyl chloride (2.88 g, 22.7 mmol) was added to the reaction vessel. After dropwise addition, the reaction was maintained at 0°C for about 2 h. The mixture was then warmed to 15°C, and purified water (50 mL, 12.5 v/v) was added to induce crystallization at 20°C. After maintaining at 20°C for 2.0 h, the mixture was filtered and the filter cake was transferred to a reaction vessel containing dichloromethane (64 mL, 16 v/v) and DBU (3.71 g, 24.4 mmol), where it was heated to reflux for 6 h. The reaction mixture was cooled to 20°C, and the pH was adjusted to 7 with 1 N hydrochloric acid. The layers were separated, and the organic phase was concentrated to dryness. Tetrahydrofuran (64 mL, 16 v/v) was added for dissolution. The solution was cooled to 20°C, and purified water (100 mL, 25 v/v) was added dropwise to induce crystallization. After maintaining at 20°C for 2.0 h, filtration was performed, and the filter cake was dried under reduced pressure to obtain compound V (3.55 g, 85% yield, 99.8% purity).

### Embodiment 3'. Preparation of compound V

Hydrochloride salt of compound IV (equivalent to 8.00 g of free base, 21.45 mmol), tetrahydrofuran (128 mL, 16 v/v), and purified water (117 mL, 14.6 v/v) were added to a reaction vessel. Mechanical stirring was started, and sodium bicarbonate (9.52 g, 113.30 mmol) was added in portions. The mixture was cooled to 2°C, and a pre-prepared solution of acryloyl chloride (4.08 g, 42.45 mmol) was added to the reaction vessel. After dropwise addition, the reaction was maintained at 2°C for about 2 h. The mixture was then warmed to 20°C, and purified water (93 mL, 11.6 v/v) was added dropwise to induce crystallization at 20°C. After maintaining at 20°C for 2.0 h, filtration was performed and the filter cake was transferred to a reaction vessel. Tetrahydrofuran (112 mL, 14 v/v) and butylated hydroxytoluene (BHT, CAS No. 128-37-0) (72 mg, 0.01 % w/w) were added. The mixture was maintained at 20°C and stirred for 1 h. Purified water (231 mL, 28.9 v/v) was added dropwise to induce crystallization at 20°C. After maintaining at 20°C for 2.0 h, the solid was filtered and dried at 25°C to obtain the dry product. The above dry product was added to a reaction vessel, followed by ethanol (56 mL, 7 v/v) and BHT (72 mg, 0.005 % w/w). The mixture was heated to 73°C and then gradually cooled to 5°C to induce crystallization. After maintaining at 5°C for 2.0 h, filtration was performed, and the filter cake was dried under reduced pressure to obtain compound V (7.34 g, 80% yield, 99.9% purity).

Embodiments 4-5 are shown in Scheme 2.

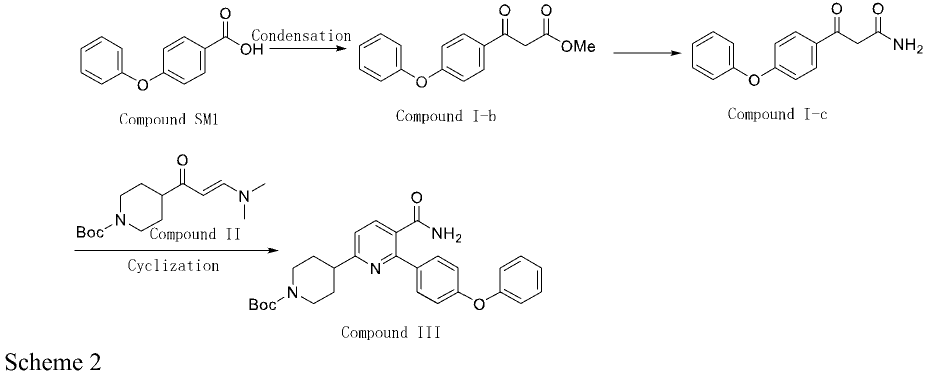

### Embodiment 4.Preparation of compound I-c

4-Phenoxybenzoic acid SM1 (4.0 g, 18.7 mmol), toluene (40 mL, 10 v/v), thionyl chloride (2.7 g, 22.7 mmol), and N,N-dimethylformamide (DMF, 5 wt%, 20 mg) were added to a reaction vessel and stirred at 60 C for 4 h. The mixture was cooled to room temperature, concentrated under reduced pressure to remove toluene. Toluene (30 mL, 7.5 v/v) was then added, and the mixture was subjected to azeotropic distillation once. Dichloromethane (30 mL, 7.5 v/v) was then added to dissolve the acyl chloride concentrate. In another reaction vessel, diisopropyl malonate (2.7 g, 18.7 mmol), 4-dimethylaminopyridine (DMAP, 2.8 g, 22.9 mmol), and dichloromethane (30 mL, 7.5 v/v) were added and cooled to 0°C. The acyl chloride solution prepared above was added dropwise, and after addition, the mixture was allowed to warm to room temperature and stirred for 16 h. The reaction was quenched with 2 N hydrochloric acid solution (60 mL, 15 v/v) and stirred for 10 min. The layers were separated, and the aqueous phase was extracted with dichloromethane (50 mL, 12.5 v/v). The organic phases were combined and sequentially washed with 2 N hydrochloric acid solution (40 mL, 10 v/v) and purified water (50 mL, 12.5 v/v), dried over anhydrous sodium sulfate, and concentrated under reduced pressure to dryness to give intermediate compound I-b (5.53 g).
MS (ESI) m/z: 271.1 (M+H⁺)

Intermediate compound I-b (5.10 g), methanol (15 mL, 7.5 v/v), and 7 N ammonia in methanol solution (25 mL, 10 v/v) were added to a reaction vessel and stirred at 40°C for 23 h. The mixture was concentrated under reduced pressure to dryness to give a crude product, which was purified to obtain compound I-c (2.7 g, 56% yield).
MS (ESI) m/z: 256.2 (M+H⁺)
¹H NMR (400 MHz, DMSO-*d*₆) δ ppm 3.32 (s, 2 H), 3.81 (s, 1 H), 7.01 - 7.17 (m, 4 H), 7.18 - 7.31 (m, 1 H), 7.40 - 7.51 (m, 2 H), 7.57 (br s, 0.6 H), 7.68 - 7.75 (m, 0.4 H), 7.95 - 8.03 (m, 1 H).

### Embodiment 5.Preparation of compound III

Compound I-c (0.50 g, 2.0 mmol), compound II (0.61 g, 2.2 mmol), ammonium acetate (0.60 g, 7.9 mmol), trifluoroethanol (15 mL, 30 v/v), and acetic acid (0.11 g, 1.8 mmol) were added to a reaction vessel and heated to 80°C with stirring for 22 h. The mixture was concentrated under reduced pressure to dryness to give a crude product, which was purified to obtain compound III (0.6 g, 65% yield).

Embodiments 6-8 are shown in Scheme 3.

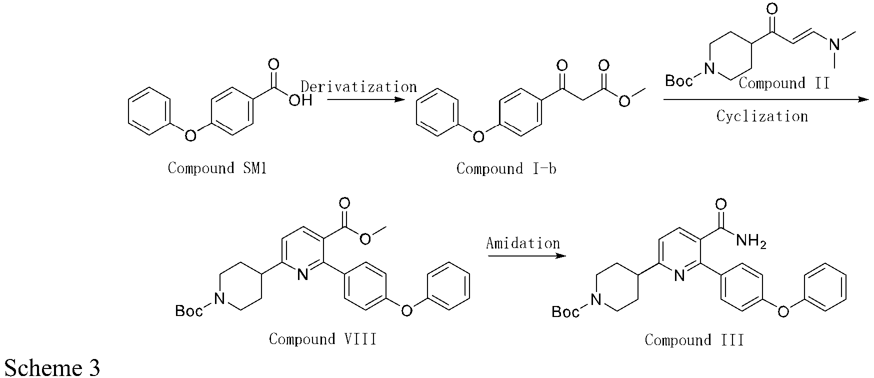

### Embodiment 6. Preparation of compound I-b

In reaction vessel A, potassium monomethyl malonate (17.5 g, 112 mmol), magnesium chloride (10.7 g, 112 mmol), and tetrahydrofuran (120 mL, 16 v/v) were added, and the mixture was heated to 65°C for the reaction. In reaction vessel B, 4-phenoxybenzoic acid SM1 (8.0 g, 37 mmol) and tetrahydrofuran (80 mL, 10 v/v) were added, and N,N'-carbonyldiimidazole (7.3 g, 45 mmol) was added in portions. The mixture was stirred at 40°C. After 3 h of reaction, the material in reaction vessel A was cooled to 20°C, and the reaction solution from reaction vessel B was added dropwise to reaction vessel A within 30 min. The resulting mixture was stirred at room temperature for 16 h. A 2 N hydrochloric acid solution (80 mL, 10 v/v) was added to quench the reaction, followed by extraction with ethyl acetate (100 mL, 12.5 v/v). The organic phase was washed with a 5 wt% sodium bicarbonate solution (80 mL, 10 v/v) and concentrated under reduced pressure to dryness to obtain the crude product compound I-b (9.9 g, 97.1% yield), which was used directly in the next step.
MS (ESI) m/z: 271.1 (M+H⁺)

### Embodiment 7. Preparation of compound VIII

Compound I-b (5.7 g, 21 mmol), compound II (6.57 g, 23.3 mmol), ammonium acetate (6.5 g, 84 mmol), trifluoroethanol (30 mL, 5 v/v), and acetic acid (1.2 g, 20 mmol) were added to the reaction vessel and the mixture was heated to 80°C and stirred for 22 h. After cooling to room temperature, the mixture was concentrated under reduced pressure to remove the solvent. Ethyl acetate (100 mL, 18 v/v) and purified water (40 mL, 7 v/v) were added to the residue and stirred for 15 min, followed by phase separation. The organic layer was washed with purified water (40 mL, 7 v/v) and concentrated under reduced pressure to dryness to give a crude product. Methanol (50 mL, 9 v/v) was then added, and the mixture was heated to 60°C and stirred for 20 min, then slowly cooled to 15°C and stirred for 1 h. Filtration was performed and the filter cake was rinsed with methanol (15 mL, 3 v/v). The wet material was dried under vacuum to obtain compound VIII (8.6 g, 84% yield, 98.6% purity).
MS (ESI) m/z: 489.3 (M+H⁺)
¹H NMR (400 MHz, DMSO-*d*₆) δ ppm 1.41 (s, 9 H), 1.53 - 1.71 (m, 2 H), 1.88 (br d, *J=11.00* Hz, 2 H), 2.85 (br s, 1 H), 2.97 (tt, J=11.74, 3.55 Hz, 1 H), 3.31 (s, 1 H), 3.68 (s, 3 H), 4.07 (br d, J=12.47 Hz, 2 H), 7.02 - 7.12 (m, 4 H), 7.15 - 7.23 (m, 1 H), 7.38 (d, J=8.07 Hz, 1 H), 7.40 - 7.47 (m, 2 H), 7.49 - 7.57 (m, 2 H), 8.05 (d, J=8.07 Hz, 1 H).

### Embodiment 8. Preparation of compound III

Compound VIII (2.6 g, 5.3 mmol) and a methanolic ammonia solution (7 M, 20 mL, 8 v/v) were added to a high-pressure reaction vessel, then heated to 70°C and stirred for 8 h. After cooling to room temperature, the mixture was concentrated under reduced pressure to remove the solvent. Ethyl acetate (40 mL, 15 v/v) and purified water (20 mL, 7.5 v/v) were added to the residue and stirred for 15 min, followed by phase separation. The organic phase was concentrated under reduced pressure to dryness to give a crude product, which was purified to obtain compound III (2.0 g, 80% yield, 97.2% purity).

Embodiments 9-10 are shown in Scheme 4.

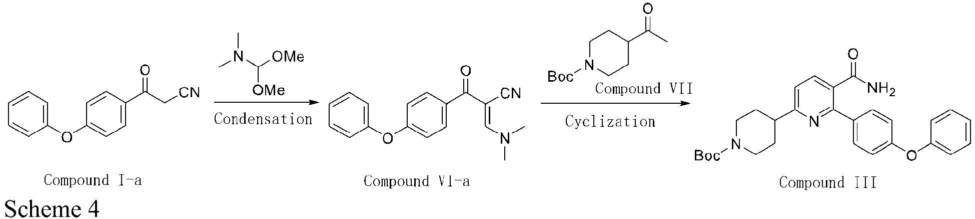

### Embodiment 9. Preparation of compound VI-a

Compound I-a (2.0 g, 8.4 mmol), 1,4-dioxane (20 mL, 10 v/v), and N,N-dimethylformamide dimethyl acetal (1.6 g, 13.4 mmol) were added to a reaction vessel and heated to 100°C for 2 h of reaction. After cooling to room temperature, the reaction solution was concentrated under reduced pressure. Ethanol (6 mL, 3 v/v) was added to the residue, and the mixture was stirred at room temperature for 15 min. Filtration was performed, and the filter cake was washed with n-hexane (5 mL, 2.5 v/v) and dried under vacuum to obtain compound VI-a (1.4 g, 56.1% yield).

### Embodiment 10. Preparation of compound III

Compound VI-a (3.0 g, 10 mmol), compound VII (2.6 g, 11 mmol), potassium tert-butoxide (2.3 g, 20 mmol), and tetrahydrofuran (30 mL, 10 v/v) were added to a reaction vessel and stirred at room temperature for 1 h. Acetic acid was added dropwise to adjust the pH to about 7, followed by addition of acetic acid (30 mL, 10 v/v) and ammonium acetate (3.2 g, 41 mmol). The mixture was heated to 100°C and stirred for 12 h. After cooling to room temperature, the mixture was concentrated under reduced pressure to dryness. Ethyl acetate (50 mL, 17 v/v) and purified water (30 mL, 10 v/v) were added to the residue and stirred for 15 min, followed by phase separation. The organic layer was washed with purified water (30 mL, 10 v/v) and concentrated under reduced pressure to give a crude product, which was purified to obtain compound III (1.2 g, 26% yield, 87.5% purity).

Embodiment 11 is shown as Scheme 5.

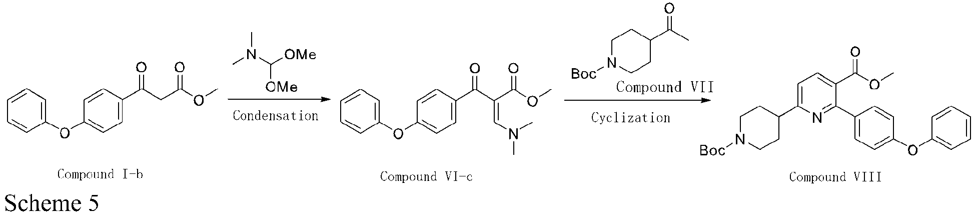

Compound I-b (1.0 g, 3.7 mmol), 1,4-dioxane (20 mL, 20 v/v), and N,N-dimethylformamide dimethyl acetal (0.5 g, 4.2 mmol) were added to a reaction vessel and heated to 100°C for 2 h of reaction. After cooling to room temperature, the reaction solution was concentrated under reduced pressure to dryness to give crude compound VI-c (0.64 g).

Compound VI-c (2.5 g, 7.7 mmol), compound VII (1.9 g, 8.4 mmol), potassium tert-butoxide (1.7 g, 15 mmol), and tetrahydrofuran (25 mL, 10 v/v) were added to a reaction vessel and stirred at room temperature for 1 h. Acetic acid was added dropwise to adjust the pH to about 7, followed by addition of acetic acid (20 mL, 8 v/v) and ammonium acetate (2.4 g, 31 mmol). The mixture was heated to 100°C and stirred for 13 h. After cooling to room temperature, the mixture was concentrated under reduced pressure to dryness. Ethyl acetate (50 mL, 20 v/v) and purified water (30 mL, 12 v/v) were added to the residue and stirred for 15 min. After phase separation, the organic layer was washed with purified water (30 mL, 12 v/v) and concentrated under reduced pressure to give a crude product, which was purified to obtain compound VIII (2.6 g, 68% yield, 98.1% purity).

## Claims

1. A method for preparing a compound of formula (III-3), 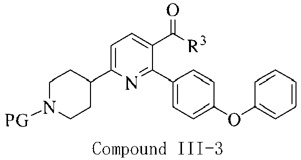 which comprises:
(a) letting a compound of formula (I) and a compound of formula (II) undergo a cyclization reaction with ammonia or an ammonium salt in the presence of an acid to obtain a compound of formula (III-3), 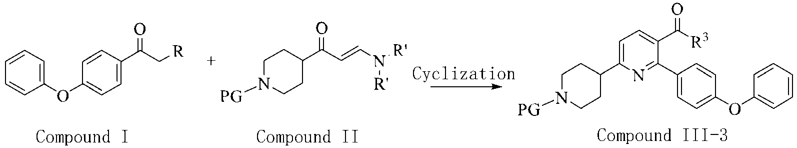 where
R is -CN, -C(O)NH₂, -C(O)N(R¹)₂, or -C(O)OR²,
R3 is -NH2, -N(R¹)₂, or -OR²,
PG represents an amino protecting group, such as acyl, alkyl, arylalkyl, alkoxycarbonyl, or arylalkoxycarbonyl groups, for example, benzyl, tert-butoxycarbonyl, benzyloxycarbonyl, fluorenylmethoxycarbonyl, or allyloxycarbonyl, preferably tert-butoxycarbonyl;
each R' is independently a substituted or unsubstituted C₁₋₆ alkyl, or R' together with the nitrogen atom to which it is attached forms a substituted or unsubstituted 3-8-membered heterocyclic group;
each R¹ is independently hydrogen or an amino protecting group, such as acyl, alkyl, arylalkyl, alkoxycarbonyl, or arylalkoxycarbonyl, for example, benzyl, tert-butoxycarbonyl, benzyloxycarbonyl, fluorenylmethoxycarbonyl, or allyloxycarbonyl, preferably tert-butoxycarbonyl; with the condition that the two R¹ groups attached to the same nitrogen atom are not simultaneously hydrogen; and
each R² is independently a substituted or unsubstituted C₁₋₆ alkyl, substituted or unsubstituted C₃-C₈ cycloalkyl, substituted or unsubstituted C₆-C₁₀ aryl, substituted or unsubstituted C₅-C₁₀ heteroaryl, substituted or unsubstituted 3-12-membered heterocyclic group, substituted or unsubstituted C₃-C₈ cycloalkyl-C₁₋₆ alkyl, substituted or unsubstituted C₆-C₁₀ aryl-C₁₋₆ alkyl, substituted or unsubstituted C₅-C₁₀ heteroaryl-C₁₋₆ alkyl, or substituted or unsubstituted 3-12-membered heterocyclic-C₁₋₆ alkyl group;
or
(b) letting a compound of formula (VI) and a compound of formula (VII) undergo a cyclization reaction with ammonia or an ammonium salt in the presence of an acid to obtain a compound of formula (III-3), 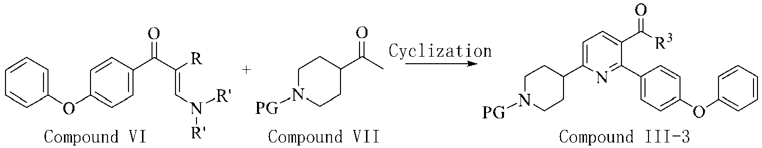 where R, R¹, R², R³, PG, and R' are as defined above.

2. The method according to Claim 1, for preparing a compound of formula (III). 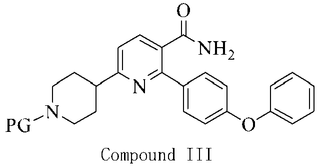 which comprises:
(A) first performing the cyclization reaction of step (a) as described in Claim 1, followed by:
(A1) when R is -CN or -C(O)NH₂, the cyclization reaction directly affords a compound of formula (III), with the compound of formula (III) corresponding to a compound of formula (III-3) in which R³ is -NH₂, 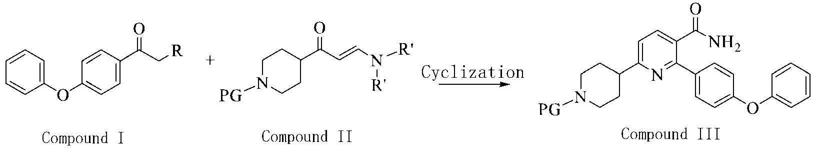
(A2) when R is -C(O)OR², after the cyclization reaction, the resulting ester intermediate III' can be converted to a compound of formula (III) via aminolysis with an amidation reagent, or the ester intermediate III' can first be hydrolyzed in a basic solution to the corresponding acid, followed by condensation with ammonia or an ammonium salt to obtain a compound of formula (III), 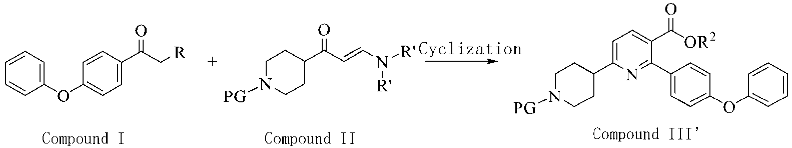 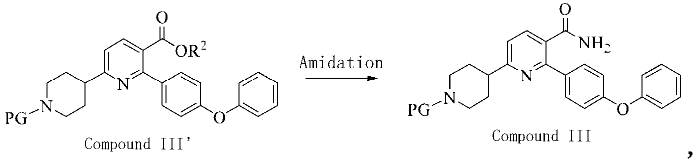 or
(A3) when R is -C(O)N(R¹)₂, after the cyclization reaction, the compound III" is converted to a compound of formula (III) via a deprotection reaction; 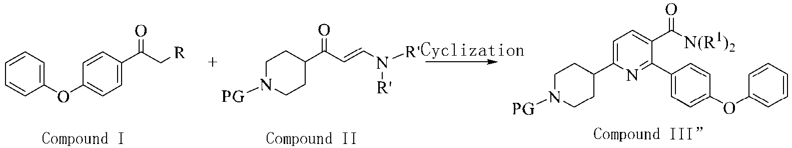 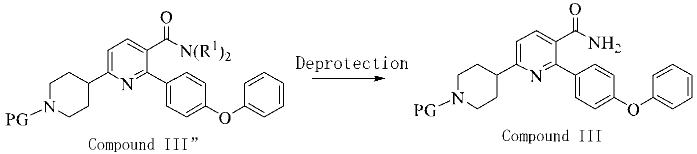 where R, R¹, R², R³, PG, and R' are as defined in Claim 1; or
(B) first performing the cyclization reaction of step (b) as described in Claim 1, followed by:
(B1) when R is -CN or -C(O)NH₂, the cyclization reaction directly affords a compound of formula (III), with the compound of formula (III) corresponding to a compound of formula (III-3) in which R³ is -NH₂, 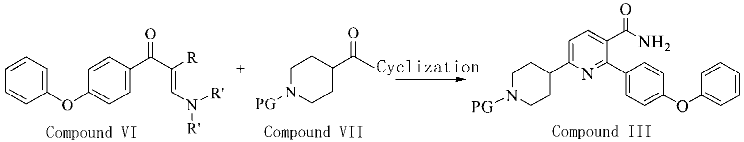
(B2) when R is -C(O)OR², after the cyclization reaction, the resulting ester intermediate III' can be converted to a compound of formula (III) via aminolysis with an amidation reagent, or the ester intermediate III' can first be hydrolyzed in a basic solution to the corresponding acid, followed by condensation with ammonia or an ammonium salt to obtain a compound of formula (III), 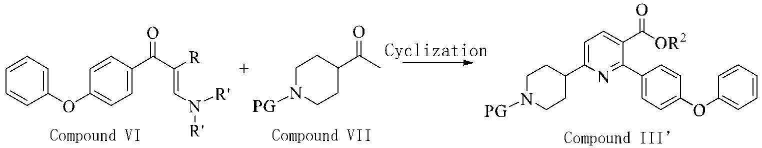 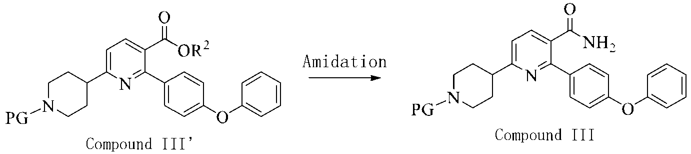
(B3) when R is -C(O)N(R¹)₂, after the cyclization reaction, the compound III" is converted to a compound of formula (III) via a deprotection reaction; 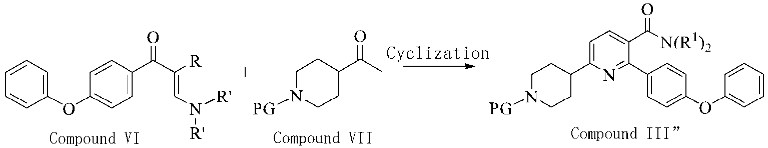 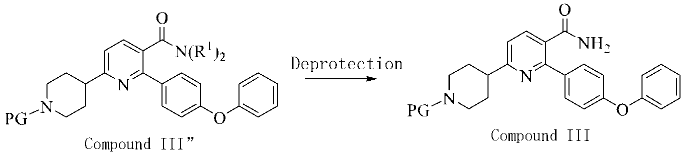 where R, R¹, R², R³, PG, and R' are as defined in Claim 1.

3. The method for preparing a compound of formula (V), which comprises:
Step 1: performing step (A) or (B) as described in Claim 2 to obtain a compound of formula (III);
Step 2: deprotecting the compound of formula (III) to obtain a compound of formula (IV); 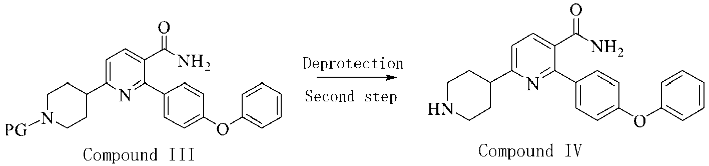 and
Step 3: letting the compound of formula (IV) undergo a reaction with acryloyl chloride, or with 3-chloropropionyl chloride followed by HCl elimination under basic conditions, thereby obtaining a compound of formula (V). 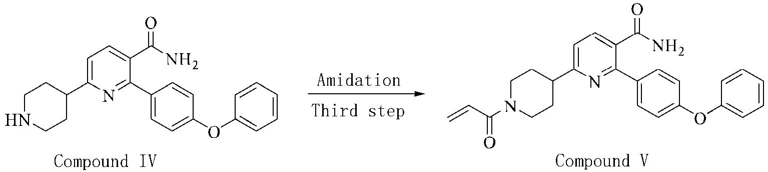 where R, R¹, R², PG, and R' are as defined in Claim 1.

4. The method for preparing a compound of formula (V), which comprises:
Step 1: performing step (A) or (B) as described in Claim 2 to obtain a compound of formula (III");
Step 2: removing the PG and R¹ groups from the compound of formula (III") to obtain a compound of formula (IV), 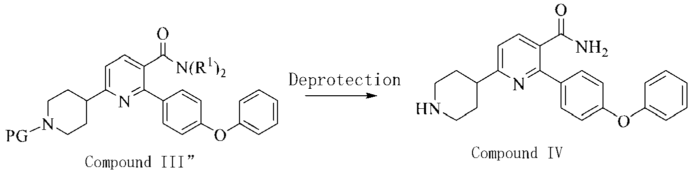 and
Step 3: letting the compound of formula (IV) undergo a reaction with acryloyl chloride, or with 3-chloropropionyl chloride followed by HCl elimination under basic conditions, thereby obtaining a compound of formula (V). 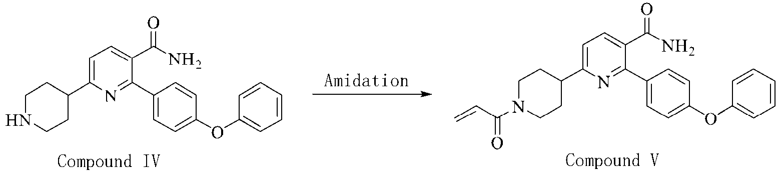
Alternatively, after step 1, perform steps 2', 3', and 4' as follows:
Step 2': deprotecting the compound of formula (III") to obtain a compound of formula (IV'); 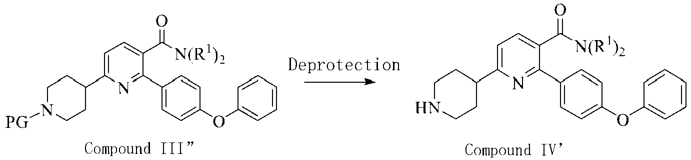
Step 3': letting the compound of formula (IV') undergo a reaction with acryloyl chloride, or with 3-chloropropionyl chloride followed by HCl elimination under basic conditions, thereby obtaining a compound of formula (V'), 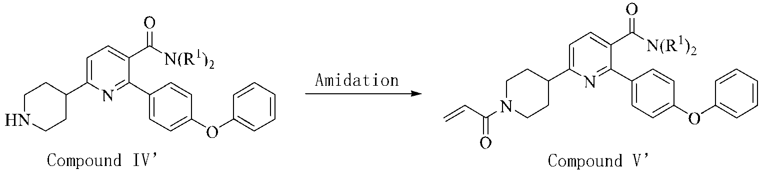
Step 4': removing the R¹ group from the compound of formula (V') to obtain a compound of formula (V), 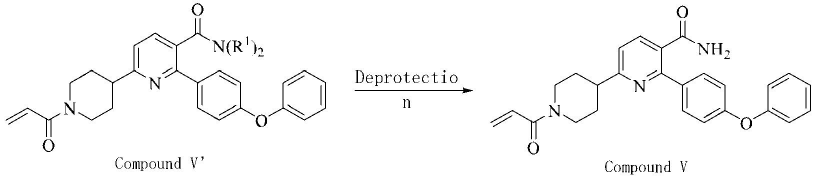 where R, R¹, R², PG, and R' are as defined in Claim 1.

5. The method for preparing a compound of formula (V), which comprises:
Step 1": performing step (A) or (B) as described in Claim 2 to obtain a compound of formula (III');
Step 2": removing the PG group from the compound of formula (III') to obtain a compound of formula (IV"), 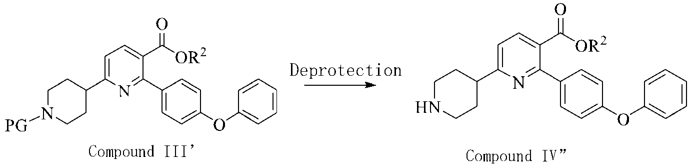
Step 3": letting the compound of formula (IV") undergo a reaction with acryloyl chloride, or with 3-chloropropionyl chloride followed by HCl elimination under basic conditions, thereby obtaining a compound of formula (V"), 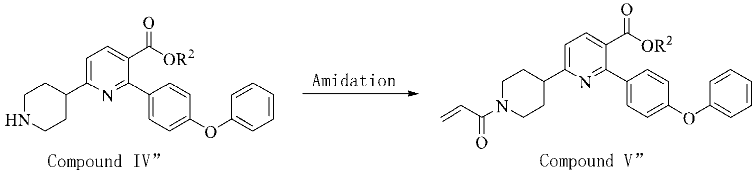
Step 4": amidation of the -OR² group of the compound of formula (V") to obtain a compound of formula (V), 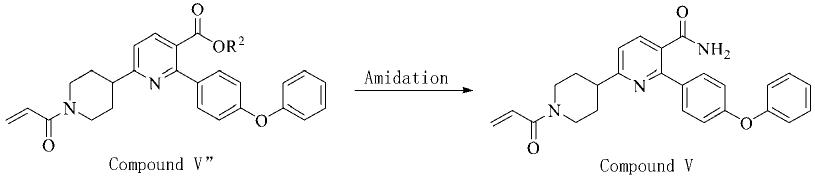 where R, R¹, R², PG, and R' are as defined in Claim 1.

6. A method for preparing a compound of formula (V), which comprises the following steps: 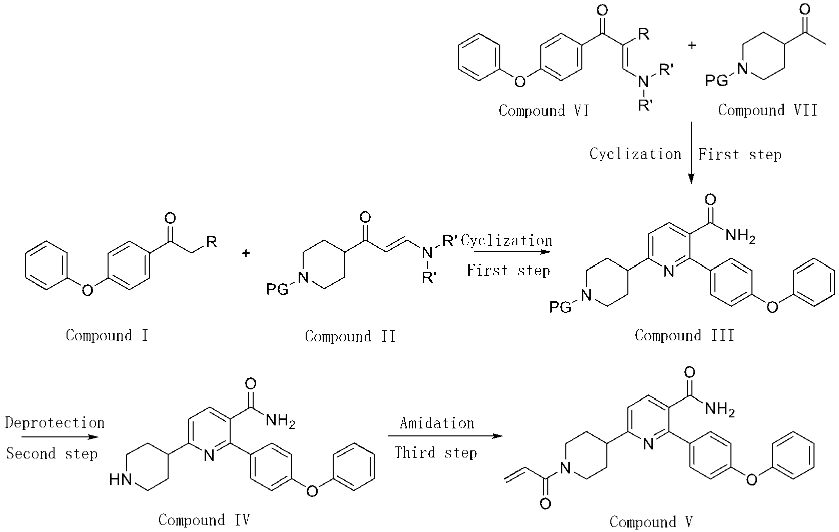
where R, R¹, R², PG, and R' are as defined in Claim 1, and
wherein reaction conditions for the first, second, and third steps are as defined in any one of Claims 1-5 for the corresponding reactions.

7. The method according to any one of Claims 1-6, wherein R is -CN, -C(O)NH₂, or - C(O)OR².

8. The method according to any one of Claims 1-7, wherein PG is selected from tert-butoxycarbonyl, benzyl, or benzyloxycarbonyl.

9. The method according to any one of Claims 1-8, wherein each R' is independently a substituted or unsubstituted C₁₋₆ alkyl.

10. The method according to any one of Claims 1-9, wherein the ammonium salt in step (a) or (b) comprises an ammonium salt formed with an inorganic or organic acid, for example, ammonium formate, ammonium acetate, ammonium oxalate, ammonium chloride, ammonium sulfate, ammonium nitrate, ammonium methanesulfonate, or ammonium trifluoroacetate.

11. The method according to any one of Claims 1-10, wherein the acid in step (a) or (b) comprises an organic or inorganic acid, for example, formic acid, acetic acid, trifluoroacetic acid, hydrochloric acid, sulfuric acid, methanesulfonic acid, oxalic acid, or p-toluenesulfonic acid.

12. The method according to any one of Claims 1-11, wherein the molar ratio of the ammonium salt to the acid in step (a) or (b) is from 0.80/1.00 to 1.20/0.80, preferably 0.95/1.00, 1.00/1.05, or 1.00/1.15, and more preferably 1.00/1.05 or 1.00/1.15.

13. The method according to any one of Claims 1-12, wherein the amidation reagent used for the -OR² group comprises ammonia or an ammonium salt, for example, ammonium chloride, ammonium formate, ammonium acetate, ammonium oxalate, ammonium sulfate, ammonium nitrate, ammonium methanesulfonate, or ammonium trifluoroacetate.

14. The method according to any one of Claims 1-13, wherein the solvent for the reaction in step (a) or (b) is selected from methanol, ethanol, trifluoroethanol, n-propanol, isopropanol, hexafluoroisopropanol, n-butanol, acetonitrile, tetrahydrofuran, toluene, or any mixture of two or more of these solvents, preferably ethanol, and more preferably a mixture of ethanol and acetonitrile.

15. The method according to any one of Claims 1-14, wherein the reaction temperature in step (a) or (b) is from 40 to 120°C, preferably from 60 to 90°C, for example, from 60 to 75°C or from 75 to 90°C, and more preferably 75°C.
